# EUROPÄISCHE PATENTSCHRIFT

(11) **EP 2 845 550 B1**
(45) Veröffentlichungstag und Bekanntmachung des Hinweises auf die Patenterteilung: **29.03.2017**
(21) Anmeldenummer: 13184193.4
(22) Anmeldetag: 12.09.2013
(51) Int. Cl.: A61B 17/295, A61B 18/14

(54) **Chirurgisches Instrument mit Kunststoffschaft**
Surgical instrument with plastic shaft
Instrument chirurgical doté d'une tige en matière plastique

(30) Priorität: 10.09.2013 EP 13183642
(43) Veröffentlichungstag der Anmeldung: 11.03.2015
(73) Patentinhaber: Erbe Elektromedizin GmbH, 72072 Tübingen (DE)
(72) Erfinder: Guba, Joachim, 71263 Weil der Stadt (DE); Mitzlaff, Lothar, 8600-531 Lagos (PT)
(74) Vertreter: Rüger, Barthelt & Abel

(56) Entgegenhaltungen:
- EP-A1- 2 294 998
- EP-A2- 2 574 299
- WO-A2-2012/151493
- US-A1- 2011 034 918
- US-A1- 2011 251 612
- US-A1- 2013 066 303

## Beschreibung

Die Erfindung betrifft ein chirurgisches Instrument, insbesondere ein elektrochirurgisches Instrument mit einem länglichen Schaft, durch den sich Zug /Schub- und Schiebeelemente zur Betätigung eines am distalen Ende des Schafts angeordneten Werkzeugs erstrecken.

Im Folgenden beschreibt der Begriff "distal" stets den vom Anwender entfernten Teil des Instruments oder Bauteils, der Begriff "proximal" beschreibt stets den zum Anwender hin gerichteten näher liegenden Teil des Instruments oder Bauteils.

Aus der EP 2 574 299 A2 ist ein chirurgisches Instrument zum Koagulieren, Versiegeln und Durchtrennen von biologischem Gewebe, wie beispielsweise Blutgefäßen, bekannt. Das Instrument trägt ein Werkzeug mit einer beweglichen und einer festen Branche auf, um Gewebe zu fassen und festzuklemmen, sowie durch Bestromung zu koagulieren. Ein linear bewegliches Messer dient zum Durchtrennen des versiegelten Gewebes. Das Werkzeug ist an dem distalen Ende eines länglichen Schafts angeordnet, dessen proximales Ende an einem Gehäuse mit Griff gehalten ist. In dem Gehäuse ist ein Betätigungsgetriebe für das Werkzeug angeordnet. Das Betätigungsgetriebe überträgt die Bewegung eines Betätigungshebels über ein Gestänge auf das Werkzeug. Das Gestänge erstreckt sich durch den Schaft. Das Gestänge wird durch einen im Querschnitt u-Profil-förmige Antriebsstange und eine darin angeordnete Messerstange gebildet.

Die EP 2 371 316 A1 offenbart ein chirurgisches Instrument zum Koagulieren, Versiegeln und Durchtrennen von biologischem Gewebe, wie beispielsweise Blutgefäßen. Das Instrument weist ein Werkzeug mit einer beweglichen Branche auf, die auf eine andere Branche hin beweglich ist, um das Gewebe zu fassen und festzuklemmen, sowie zu bestromen. Zu dem Werkzeug gehört außerdem ein Messer, das linear beweglich ist, um das versiegelte Gewebe zu durchtrennen. Das Werkzeug ist an dem distalen Ende eines länglichen Schafts angeordnet, dessen proximales Ende an einem Gehäuse gehalten ist. Das Gehäuse schließt ein Betätigungsgetriebe für das Werkzeug ein und weist einen Handgriff sowie einen Betätigungshebel auf. Das Betätigungsgetriebe überträgt die Bewegung des Betätigungshebels auf das Werkzeug, um die Branchen zu schließen und das Messer vorzuschieben. Die von dem Betätigungsgetriebe umgesetzte Bewegung wird durch Zug /Schub- und Schiebeelemente auf das Werkzeug übertragen, die sich durch den Schaft erstrecken.

Damit das Messer zuverlässig einen geraden Schnitt vollführt, muss es mittels des sich durch den Schaft erstreckenden Schiebeelements sicher betätigbar sein. Dazu muss das Schiebeelement in dem Schaft geführt sein, damit es bei einer Schubbewegung nicht seitlich ausweicht. Eine Führung benötigt jedoch Spiel, damit das Schiebeelement leichtgängig zu betätigen ist. Weicht das Schiebeelement der Schubbewegung seitlich aus, kann es in der Führung zur erhöhten Reibung und zu einer unpräzisen Messerbewegung kommen.

Die US 2013/0066303 A1 beschreibt dazu ein Instrument mit einer Antriebsstange, die einen längs verlaufenden Schlitz aufweist. In diesem ist ein streifenförmiges Schubelement angeordnet, dessen Stirnkante als Messer dient.

Es ist Aufgabe der Erfindung, ein unabhängig von der Schaftlänge zuverlässig arbeitendes chirurgisches Instrument mit mindestens einer beweglichen Branche und mindestens einem linear beweglichen Messer zu schaffen.

Diese Aufgabe wird mit dem Instrument nach Anspruch 1 gelöst.

Das chirurgische Instrument weist ein Werkzeug mit zwei Branchen auf, von denen wenigstens eine schwenkbeweglich gelagert ist. Die Branchen sind vorzugsweise mit Elektrodeneinheiten versehen, die elektrisch leitfähige Gewebekontaktflächen aufweisen, die als Elektroden dienen und zwischen denen biologisches Gewebe, insbesondere Blutgefäße zu fassen und zusammenzudrücken sind. Die Gewebekontaktflächen der Elektrodeneinheiten sind mit einer elektrischen Quelle, beispielsweise einem HF-Generator verbunden oder verbindbar. Sie können über einen vorzugsweise am Gehäuse des Instruments angeordneten Schalter gezielt bestromt werden, um dazwischen gefasstes biologisches Gewebe zu koagulieren oder zu versiegeln.

Zu dem Werkzeug gehört außerdem ein Messer, das in einer geeigneten, in den Branchen vorgesehenen Messerführungsnut in distaler Richtung schiebend bewegt werden kann, um ein zwischen den Elektrodeneinheiten der Branchen gefasstes Gewebe zu durchtrennen.

Das Werkzeug ist an einem länglichen gerade ausgebildeten Schaft gehalten, der sich von dem Gehäuse weg erstreckt. Der Schaft weist in seiner Längsrichtung eine Öffnung auf und umschließt damit einen Kanal, durch den sich mindestens ein Element erstreckt, um wenigstens einer der Branchen eine Bewegung zum Schließen bzw. Öffnen erteilt. Dieses Element, ein Zug-/Schubelement wird auf Schub beim Schließen und auf Zug beim Öffnen einer Branche beansprucht. Wenn erfindungsgemäss beide Branchen beweglich sind, erstrecken sich zwei Zug-/Schubelemente durch den Kanal des Schafts um die Branchen aufeinander hin zu bewegen, wenn die Zug-/Schubelemente ziehend bewegt werden. Die sich im Inneren des Schafts erstreckende Öffnung, der Kanal, kann unterschiedlich beispielsweise zylindrisch, konisch oder anders geformt sein.

Das Messer wird durch ein Schiebeelement betätigt. Dieses ist zwischen mindestens einem Zug-/Schubelement und einem Widerlager angeordnet und erstreckt sich parallel zu dem Zug-/Schubelement durch den Schaft. Das Widerlager kann eine Fläche des Schafts oder, erfindungsgemäss, ein zweites Zug-/Schubelement sein. Diese Anordnung nutzt den Umstand, dass das Messer nur dann schiebend in distaler Richtung zu bewegen ist, wenn die beiden Branchen fest gegeneinander drücken und gegebenenfalls Gewebe, vorzugsweise koaguliertes Gewebe zwischen den Elektrodeneinheiten eingeklemmt halten. In diesem Zustand sind die beiden Zug-/Schubelement straff gespannt, um der Branche oder den Branchen das nötige Drehmoment zum Schließen zu verleihen. In diesem Zustand kann das Schiebeelement zwischen den Zug-/Schubelementen knickfrei geführt werden. Dies schafft die Möglichkeit, das Schiebeelement als flaches streifenförmiges Element, beispielsweise als Metallband, auszubilden, das zum Beispiel im Wesentlichen zentrisch durch den Schaft läuft und an seinen beiden Flachseiten durch die gespannten Zug-/Schubelemente abgestützt wird. Diese stehen unter Zugspannung und können das Schiebeelement deswegen spiel- und reibungsarm präzise führen.

Die Zug-/Schubelemente sind draht- oder streifenförmig ausgebildet. Vorzugsweise bestehen sie aus Metall. Auch das Schiebeelement besteht vorzugsweise aus Metall. Es kann mit dem Messer einteilig ausgebildet sein.

Das Messer ist vorzugsweise in einem Sockelteil linear beweglich geführt. Das Sockelteil kann außerdem dazu dienen, eine oder beide Branchen schwenkbeweglich aufzunehmen.
Es wird an einem Ende des Schafts angeordnet. Das Sockelteil und der Schaft können einstückig, nahtlos ausgebildet sein. Vorzugsweise ist an dem gegenüberliegenden proximalen Ende des Schafts eine Rohrendhülse angeordnet, die eine Parallelführung von Schiebeelement und Zug-/Schubelement (en) erbringt. Damit sind die Schiebe- und Zugelemente an beiden Enden des Schafts präzise geführt. Die Rohrendhülse kann einstückig, nahtlos mit dem Schaft verbunden sein.

Weitere Einzelheiten vorteilhafter Ausführungsformen und Details der Erfindung sind Gegenstand der Beschreibung der Zeichnung oder von Ansprüchen. Es zeigen:
Figur 1 das erfindungsgemäße Instrument, in perspektivischer Prinzipdarstellung,
Figur 2 das Werkzeug des Instruments nach Figur 1, in perspektivischer Darstellung,
Figur 3 ein zu dem Werkzeug nach Figur 2 gehöriges Sockelteil, in perspektivischer Darstellung,
Figur 4 eine Branche und ihr zugehöriges Zug-/Schubelement, in perspektivischer Darstellung,
Figur 5 Zug-/Schub- - und Schiebeelemente für das Werkzeug nach Figur 2, in perspektivischer Explosionsdarstellung,
Figur 6 die Zug-/Schub- - und Schiebeelemente nach Figur 5, in Querschnittsansicht und Einbauposition,
Figur 7 das Werkzeug mit Schaft und Zug-/Schub - und Schiebeelementen in perspektivischer Prinzipdarstellung,
Figur 8 eine Kabelführungshülse für die Zug-/Schubelemente nach Figur 7, in perspektivischer Darstellung, und
Figur 9 eine Rohrendhülse für die Anordnung nach Figur 7, in perspektivischer Darstellung.

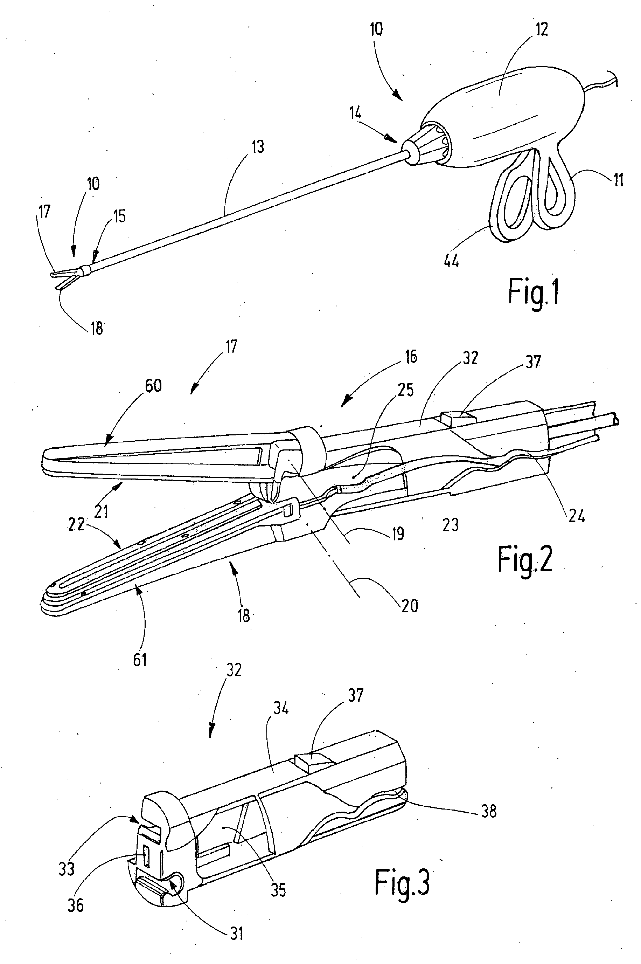

In Figur 1 ist ein chirurgisches Instrument 10 veranschaulicht, das ein mit einem Handgriff 11 versehenes Gehäuse 12 aufweist. An dem Gehäuse 12 ist ein Schaft 13 gehalten, dessen proximales Ende 14 an dem Gehäuse 12 gehalten ist. Das distale Ende 15 des Schafts 13 trägt ein Werkzeug 16 zur Einwirkung auf biologisches Material, beispielsweise zum Abklemmen, Koagulieren und Verschweißen sowie nachfolgendem Trennen von Gewebe, zum Beispiel Blutgefäßen.

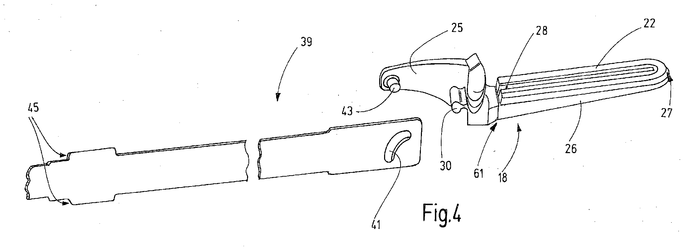

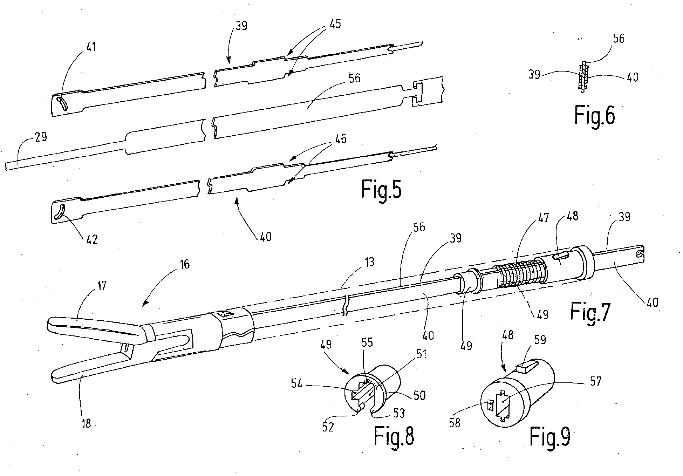

Das Werkzeug 16 ist in Figur 2 gesondert veranschaulicht. Es weist eine erste Branche 17 und eine zweite Branche 18 auf, von denen mindestens eine, im vorliegenden Ausführungsbeispiel beide Branchen 17, 18 um Scharnierachsen 19, 20 schwenkbeweglich gelagert sind. Jede Branche 17, 18 besteht aus einem Branchenträger 60, 61 und den darauf fixierten Elektrodeneinheiten 21, 22. Die Branchenträger 60, 61 dienen dabei zur Übertragung der mechanischen Kräfte und zur Lagerung der Branche 17, 18. Die Branchen 17, 18 weisen Elektrodeneinheiten 21, 22 auf, die über elektrische Leitungen mit elektrischer Energie beaufschlagbar sind. In Figur 2 ist dazu die mit der Elektrodeneinheit 22 verbundene Leitung 23 ersichtlich. Die Seele der Leitung 23 ist mit einem Blechteil der Elektrodeneinheit 22 verbunden, welches auch die Gewebekontaktfläche bildet. Die Leitung 23 ist im weiteren Verlauf isoliert und in einem gewellten Kanal 24 gehalten, der sich durch ein Betätigungsteil 25 des Branchenträgers 61 der Branche 18 erstreckt. Wie Figur 4 erkennen lässt, umfasst der Branchenträger 61 der Branche 18 einen dem Betätigungsteil 25 entgegengesetzten Werkzeugteil 26, der die Elektrodeneinheit 22 trägt. In der Elektrodeneinheit 22 ist mittig ein beidseitig isolierter Kanal oder eine Messerführungsnut 28 vorgesehen, die zur Aufnahme eines Messers 29 (Figur 5) dient. Das Messer 29 weist an seiner distalen Stirnseite quer zu seiner Bewegungsrichtung eine Schneidkante auf.

Zwischen dem Betätigungsteil 25 und dem Werkzeugteil 26 ist der Branchenträger 61 der Branche 18 mit einem Lagerzapfen 30 versehen, der in eine Lagerbuchse 31 eines Sockelteils 32 einschiebbar ist. Das vorzugsweise aus Kunststoff bestehende Sockelteil 32 weist parallel zu der Lagerbuchse 31 eine weitere Lagerbuchse 33 für die erste Branche 17 auf, die ähnlich wie die oben beschriebene zweite Branche 18 oder als Gleichteil zu dieser aufgebaut ist. Entsprechend ist auch die Elektrodeneinheit 21 der Branche 17 über eine entsprechende Leitung mit einer elektrischen Quelle verbunden.

Das Sockelteil 32 weist einen in den Schaft 13 einführbaren und mit diesem verrastbaren Fortsatz 34 auf, der ein zentrales Fenster 35 umgibt. Dieses geht zwischen den Lagerbuchsen 31, 33 in einen Schlitz 36 zur Führung des Messers 29 über.

An dem Fortsatz 34 sind ein oder mehrere Rastnasen 37 zur rastenden Befestigung an dem Schaft 13 ausgebildet. Außerdem sind an dem Fortsatz 34 an beiden Flanken gewellte Nuten 38 zur Festlegung und Zugsicherung der Leitung 23 ausgebildet.

Zur Betätigung der Branchen 17, 18, das heißt zum Schwenken derselben, dienen Zug- /Schubelemente 39, 40, wie sie aus Figur 5 ersichtlich sind. Sie bestehen vorzugsweise aus schmalen Blechstreifen mit einer distal angeordneten Kulissenführung 41, 42 zur Aufnahme jeweils eines Betätigungszapfens 43, wie er aus Figur 4 an dem Branchenträger 61 der Branche 18 ersichtlich ist. Ein ähnlicher Betätigungszapfen ist an der Branche 17 vorgesehen.

Die beiden Zug-/Schubelement 39, 40 erstrecken sich, wie Figur 7 erkennen lässt, durch den gesamten Schaft 13 in das Gehäuse 12 hinein. In diesem befindet sich ein Kraftübertragungsmittel zur Übertragung einer auf einen Bedienhebel 44 ausgeübten Betätigungsbewegung auf die Zug-/Schubelemente 39, 40.

In der Nähe ihres proximalen Endes weisen die Zug-/Schubelemente 39, 40 Absätze 45, 46 auf, die zur Abstützung einer Druckfeder 47 dienen. Die Druckfeder 47 stützt sich an ihrem anderen Ende an einer Rohrendhülse 48 ab, wie sie aus Figur 9 erkennbar ist.

Auf den Zug-/Schubelementen 39, 40 sitzt innerhalb des Schafts 13 außerdem eine Kabelführungshülse 49 gemäß Figur 8. Die Kabelführungshülse 49 ist beispielsweise als Kunststoffspritzgussteil ausgeführt. Sie ist an zumindest einer Seite der Absätze 45, 46 oder an beiden Seiten angeordnet, wie beispielsweise in der in Figur 7 veranschaulichten Position und zusätzlich innerhalb der Druckfeder 47. Die primäre Funktion der Kabelführungshülse 48 liegt darin, eine oder mehrere elektrische Leitungen, wie zum Beispiel die Leitung 23, in einer definierten Position zu halten und somit zu verhindern, dass die Leitungen in der Druckfeder 47 eingeklemmt werden.

Die in Figur 7 rechte mit gestrichelter Bezugslinie dargestellte Kabelführungshülse 49 bildet einen Anschlag für die Druckfeder 47. Dazu weist sie einen radialen Flansch oder Bund 50 auf. Die Kabelführungshülse 49 weist einen zentralen, im Querschnitt im Wesentlichen rechteckigen Kanal 51 auf, der nach unten geschlitzt ist, so dass die Kabelführungshülse 49 über die Zug-/Schubelemente 39, 40 geschoben werden kann. An dem unteren Rand des Kanals 51 weist der Bund 50 Rastnasen 52, 53 auf, um die Kabelführungshüle 49 auf den Zug-/Schubelementen 39, 40 zu halten. Seitlich und nach oben ist der Kanal 51 durch nutartige Vertiefungen 54, 55 erweitert. In der Vertiefung 54 können elektrische Leitungen oder Kabel aufgenommen sein. Die Vertiefung 55 dient zur Führung eines nachfolgend erläuterten Schiebeelements 56. Es ist nicht erforderlich, dass die Kabelführungshülsen 49 geometrisch identisch ausgebildet sind. Wichtig ist, dass die Kabelführungshülsen 49 die oben beschriebenen Merkmale aufweisen.

Das Schiebeelement 56 gehört zu dem Messer 29. Es ist mit diesem beispielsweise einstückig in Gestalt eines flachen Blechstreifens mit gleichmäßiger Dicke ausgebildet. Die Höhe (in Figur 5 vertikal zu messen) des Schiebeelements 56 ist vorzugsweise größer als die Höhe des Messers 29. Außerdem ist die Höbe des Schiebeelements 56 vorzugsweise etwas größer die Höhe der Zug-/Schubelemente 39, 40. Die Verhältnisse sind in Figur 6 verdeutlicht, die einen Schnitt an einer in dem Schaft 13 liegenden Stelle wiedergibt. Wie ersichtlich überragt das Schiebeelement 56 die Zug-/Schubelemente 39, 40 oben und/oder unten. Der obere Teil des Schiebeelements 56 kann in der Vertiefung 55 geführt werden.

Die Rohrendhülse 48 weist ebenfalls einen Kanal 57 auf, der dem in Figur 6 insgesamt dargestellten Querschnitt angepasst ist. Neben und parallel zu diesem Kanal 57 kann ein Durchgang 58 für elektrische Leitungen vorgesehen sein. Außerdem kann die Rohrendhülse 48 mit einer Rastnase 59 zur Befestigung an dem Schaft 13 vorgesehen sein.

Das in soweit beschriebene Instrument 10 arbeitet wie folgt:
In unbetätigtem Zustand sind die Branchen 17, 18 voneinander weg gespreizt und die Schneidkante des Messers 29 befindet sich innerhalb des Sockelteils 32. Die Druckfeder 47 drängt die Zug-/Schubelemente 39, 40 in distaler Richtung, wodurch die Branchen 17, 18 gespreizt bleiben.

Betätigt der Anwender nun den Bedienhebel 44, wird diese Betätigungsbewegung von einem in dem Gehäuse 12 angeordneten Getriebe in eine Zugbewegung umgesetzt, die auf die proximalen Enden der Zug-/Schubelemente 39, 40 übertragen wird. Diese Zugbewegung in proximaler Richtung schließt die Branchen 17, 18 gegen die Kraft der Druckfeder 47. Die Branchen 17, 18 fassen das zwischen ihnen liegende biologische Gewebe und halten es fest geklemmt. Das Gewebe kann mittels eines nicht weiter dargestellten Schalters und durch Aktivierung eines angeschlossenen Generators über die Elektrodeneinheiten 21, 22 bestromt und koaguliert werden.

Soll das koagulierte Gewebe nun beispielsweise getrennt werden, wird das Schiebeelement 56 von dem in dem Gehäuse 12 angeordneten Mechanismus in distaler Richtung bewegt. Das Schiebeelement 56 kann eine Länge von mehreren 100 mm beispielsweise 265 mm aufweisen, wobei es eine geringe Dicke von lediglich zum Beispiel 0,2 bis 0,3 mm aufweist. Es ist zwischen den gespannten Zug-/Schubelementen 39, 40 gehalten und kann dadurch seitlich nicht ausknicken. Es schiebt somit das seinerseits sicher in dem Messerführungsnut 28 geführte Messer 29 mit an dessen Stirnfläche versehene Schneidkante mit der nötigen Kraft durch denaturiertes Gewebe und trennt dieses mit einem sauberen Schnitt sicher.

Bei dem elektrochirurgischen Instrument 10 ist ein Werkzeug 16 vorgesehen, zu dessen Betätigung mindestens ein, vorzugsweise zwei Zug-/Schubelemente 39, 40 und ein Schiebeelement 56 erforderlich sind. Das Schiebeelement 56 ist zwischen einem Zug-/Schubelement 39 und einem Widerlager gehalten, wobei das Widerlager durch ein weiteres Zug-/Schubelement 40 gebildet sein kann. Diese Anordnung nutzt den Umstand, dass das Schiebeelement 56 nur dann aktiviert werden muss, wenn das Zug-/Schubelement 39 (40) straff gespannt ist. Dieses Konzept der Führung des Schiebeelements 56 an dem (den) Zug-/Schubelement (en) 39 (40) ermöglicht den einfachen Aufbau von Instrumenten mit langen Schäften, wobei keine gesonderten Maßnahmen zur seitlichen Aussteifung des Schiebeelements getroffen werden müssen. Zur Ausbildung des Schafts 13 kann ein Kunststoffrohr genutzt werden. Die sich in Längsrichtung des Rohrs erstreckende Öffnung, der Kanal kann unterschiedlich beispielsweise zylindrisch, konisch oder anders geformt sein.

### Bezugszeichenliste:

- 10: Instrument
- 11: Handgriff
- 12: Gehäuse
- 13: Schaft
- 14: proximales Ende des Schafts 13
- 15: distales Ende des Schafts 13
- 16: Werkzeug
- 17: erste Branche
- 18: zweite Branche
- 19: Scharnierachse der ersten Branche 17
- 20: Scharnierachse der zweiten Branche 18
- 21: Elektrodeneinheit der ersten Branche 17
- 22: Elektrodeneinheit der zweiten Branche 18
- 23: Leitung
- 24: gewellter Kanal
- 25: Betätigungsteil
- 26: Werkzeugteil
- 28: Messerführungsnut
- 29: Messer
- 30: Lagerzapfen
- 31: Lagerbuchse für die zweite Branche 18
- 32: Sockelteil
- 33: Lagerbuchse für die erste Branche 17
- 34: Fortsatz
- 35: Fenster
- 36: Schlitz
- 37: Rastnase
- 38: Nut
- 39, 40: Zug / Schubelement
- 41, 42: Kulissenführung
- 43: Betätigungszapfen
- 44: Bedienhebel
- 45, 46: Absätze
- 47: Druckfeder
- 48: Rohrendhülse
- 49: Kabelführungshülse
- 50: Bund
- 51: Kanal
- 52, 53: Rastnasen
- 54, 55: Vertiefungen
- 56: Schiebeelement
- 57: Kanal
- 58: Durchgang
- 59: Rastnase
- 60, 61: Branchenträger

## Patentansprüche

1. Chirurgisches Instrument (10),
mit einem Werkzeug (16), das ein schiebend bewegbares Messer (29), eine erste und eine zweite Branche (17, 18) aufweist, von denen beide schwenkbeweglich gelagert sind,
mit einem länglichen, gerade ausgebildeten Schaft (13), der ein distales Ende (15) aufweist, an dem das Werkzeug (16) gehalten ist,
mit zwei Zug-/Schubelementen (39, 40), die den Branchen (17, 18) zugeordnet sind, um die Branchen (17, 18) durch Zugbewegung in proximaler Richtung zu schließen, wobei sich die Zug-/Schubelemente (39, 40) bei geschlossenen Branchen (17, 18) straff gespannt längs durch den Schaft (13) erstrecken,
mit einem Schiebeelement (56), das sich zwischen den Zug-/Schubelementen (39, 40) durch den Schaft (13) erstreckt und zwischen den Zug-/Schubelementen bei geschlossenen Branchen (17, 18) knickfrei geführt ist **dadurch gekennzeichnet, dass** das erste und zweite Zug-/Schubelement (39, 40) ein Draht oder ein streifenförmiges Element, ein Band ist, wobei sich die Zug-/Schubelemente (39, 40) bei geschlossenen Branchen (17, 18) straff gespannt längs durch den Schaft (13) erstrecken.

2. Instrument nach Anspruch 1, **dadurch gekennzeichnet, dass** das Schiebeelement (56) zwischen den Zug-/Schubelementen (39, 40) an diesen anliegend gehalten ist, wobei die Zug-/Schubelemente (39, 40) an Flachseiten des Schiebeelements (56) anliegen.

3. Instrument nach einem der vorstehenden Ansprüche, **dadurch gekennzeichnet, dass** der Schaft (13) aus Kunststoff ausgebildet ist.

4. Instrument nach einem der vorstehenden Ansprüche, **dadurch gekennzeichnet, dass** der Schaft (13) einen sich in Längsrichtung erstreckenden konischen Kanal aufweist.

5. Instrument nach einem der vorstehenden Ansprüche, **dadurch gekennzeichnet, dass** das Schiebeelement (56) mit dem Messer (29) einteilig ausgebildet ist.

6. Instrument nach einem der vorstehenden Ansprüche, **dadurch gekennzeichnet, dass** das Messer (29) in einem Sockelteil (32) linearbeweglich geführt ist.

7. Instrument nach Anspruch 6, **dadurch gekennzeichnet, dass** das Sockelteil (32) aus Kunststoff ausgebildet ist.

8. Instrument nach Anspruch 6 oder 7, **dadurch gekennzeichnet, dass** die Branche (17, 18) an dem Sockelteil (32) gelagert ist.

9. Instrument nach einem der Ansprüche 6 - 8, **dadurch gekennzeichnet, dass** das Sockelteil (32) mit dem Schaft 13) verrastet ist.

## Claims

1. Surgical instrument (10),
with a tool (16) having a sliding movable cutter (29), a first and a second branch (17, 18), both of which are mounted pivotably movably,
with a linear straight shaft (13) having a distal end (15) on which the tool (16) is held, with two pull/push elements (39, 40) assigned to the branches (17, 18) to close the branches (17, 18) by a pull movement in the proximal direction, wherein the pull/push elements (39, 40) extend tightly tensioned along and through the shaft (13) when the branches (17, 18) are closed,
with a slide element (56) which extends through the shaft (13) between the pull/push elements (39, 40) and is guided in kink-free fashion between the pull/push elements when the branches (17, 18) are closed,
**characterised in that** the first and second pull/push element (39, 40) is a wire or a strip-like element, a strap, wherein the pull/push elements (39, 40) extend tightly tensioned along and through the shaft (13) when the branches (17, 18) are closed.

2. Instrument according to claim 1, **characterised in that** the slide element (56) is held between the pull/push elements (39, 40) and lying thereon, wherein the pull/push elements (39, 40) lie on flat sides of the slide element (56).

3. Instrument according to any of the preceding claims, **characterised in that** the shaft (13) is made of plastic.

4. Instrument according to any of the preceding claims, **characterised in that** the shaft (13) has a conical channel extending in the longitudinal direction.

5. Instrument according to any of the preceding claims, **characterised in that** the slide element (56) is formed integrally with the cutter (29).

6. Instrument according to any of the preceding claims, **characterised in that** the cutter (29) is guided linearly movably in a base part (32).

7. Instrument according to claim 6, **characterised in that** the base part (32) is made of plastic.

8. Instrument according to claim 6 or 7, **characterised in that** the arms (17, 18) are mounted on the base part (32).

9. Instrument according to any of claims 6 to 8, **characterised in that** the base part (32) is locked to the shaft (13).

## Revendications

1. Instrument chirurgical (10),
comprenant un outil (16) qui présente un couteau (29) à déplacement coulissant, un premier et un deuxième mors (17, 18), qui sont tous deux montés avec possibilité de pivotement,
comprenant une tige (13) allongée, réalisée sous une forme rectiligne, qui présente une extrémité distale (15) sur laquelle est tenu l'outi (16),
comprenant deux éléments de traction/poussée (39, 40) qui sont associés aux mors (17, 18) pour fermer les mors (17, 18) par un mouvement de traction dans la direction proximale, sachant que lorsque les mors (17, 18) sont fermés, les éléments de traction/poussée (39, 40) s'étendent dans le sens longitudinal dans la tige (13), en étant bien tendus,
comprenant un élément coulissant (56) qui s'étend dans la tige (13), entre les éléments de traction/poussée (39, 40), et est guidé de façon exempte de flexions entre les éléments de traction/poussée (39, 40) lorsque les mors (17, 18) sont fermés,
**caractérisé en ce que** le premier et le deuxième élément de traction/poussée (39, 40) est un fil métallique ou un élément en forme de bande, un ruban, les éléments de traction/poussée (39, 40) s'étendant dans le sens longitudinal dans la tige (13), en étant bien tendus, lorsque les mors (17, 18) sont fermés.

2. Instrument selon la revendication 1, **caractérisé en ce que** l'élément coulissant (56) est tenu entre les éléments de traction/poussée (39, 40) en étant appliqué contre ceux-ci, les éléments de traction/poussée (39, 40) étant appliqués contre les grands côtés de l'élément coulissant (56).

3. Instrument selon l'une des revendications précédentes, **caractérisé en ce que** la tige (13) est réalisée en matière plastique.

4. Instrument selon l'une des revendications précédentes, **caractérisé en ce que** la tige (13) présente un canal conique s'étendant dans le sens longitudinal.

5. Instrument selon l'une des revendications précédentes, **caractérisé en ce que** l'élément coulissant (56) est réalisé d'une seule pièce avec le couteau (29).

6. Instrument selon l'une des revendications précédentes, **caractérisé en ce que** le couteau (29) est guidé avec possibilité de déplacement linéaire dans un élément formant socle (32).

7. Instrument selon la revendication 6, **caractérisé en ce que** l'élément formant socle (32) est réalisé en matière plastique.

8. Instrument selon la revendication 6 ou 7, **caractérisé en ce que** le mors (17, 18) est monté sur l'élément formant socle (32).

9. Instrument selon l'une des revendications 6 à 8, **caractérisé en ce que** l'élément formant socle (32) est encliqueté avec la tige (13).
